# EUROPEAN PATENT APPLICATION

(11) **EP 3 868 395 A1**
(43) Date of publication of application: **25.08.2021**
(21) Application number: 20158912.4
(22) Date of filing: 23.02.2020
(51) Int. Cl.: A61K 38/34, A61P 25/00

(54) **TREATMENT OF MEDICAL INDICATION**

(71) Applicant: Vallaurix Trading Sarl MC, 98000 Monaco (MC)
(72) Inventor: WOLGEN, Philippe, 98000 MONACO (MC)
(74) Representative: Farago-Schauer, Peter Andreas

(57) **Abstract**

The present invention is directed to afamelanotide or a pharmaceutically acceptable salt thereof for use in treatment of a human subject suffering from cerebrovascular accident (CVA).

## Description

### Technical field

The present invention relates to alpha-MSH analogues for use in the treatment of a human subject suffering from a cerebrovascular accident (CVA).

### Background to the invention

There is a high medical need for improvement of treatment outcome of CVA in human subjects. While animal model may be used to evaluate candidates for treatment, the large number of failures of pharmaceutical agents in subsequent clinical development of for instance CVA proves that these animal models have limited value in predicting effective medication in the treatment of human subjects. This represents a major complication for development of new medication for treatment of CVA.

Accordingly, there is a high unmet medical need to improve the treatment of CVA in humans with better clinical outcomes.

### Summary of the invention

The present invention is directed to a compound for use in the treatment of a human subject suffering from cerebrovascular accident (CVA) wherein the compound is afamelanotide or a pharmaceutically acceptable salt thereof.

Preferably, afamelanotide or a pharmaceutically acceptable salt thereof is administered in the form of an injectable preparation or dosage form. Preferably, afamelanotide or a pharmaceutically acceptable salt thereof is administered in an immediate or controlled release injection. Preferably, afamelanotide or a pharmaceutically acceptable salt thereof is injected subcutaneously. Preferably, afamelanotide or a pharmaceutically acceptable salt thereof is administered with a dose of from 0.1 to 1mg/kg/day for at least 1 day, wherein kg represents the weight of the human subject in kilograms. Preferably, afamelanotide or a pharmaceutically acceptable salt thereof is administered at a dose of from 20 to 100mg. In one preferred embodiment, the invention relates to ischemic CVA. In another preferred embodiment, the invention relates to hemorrhagic CVA. Preferably, the human subject is treated with a tissue plasminogen activator (tPA) and subsequently or prior treated with afamelanotide or a pharmaceutically acceptable salt thereof.

In a further embodiment, the invention is directed to a composition comprising afamelanotide or a pharmaceutically acceptable salt thereof for use in treatment of a human subject suffering from cerebrovascular accident (CVA).

In a further embodiment, the invention is directed to a method of treatment of a human subject suffering from cerebrovascular accident (CVA) wherein the human subject is treated with afamelanotide or a pharmaceutically acceptable salt thereof.

In a further embodiment, the invention is directed to the use of afamelanotide or a pharmaceutically acceptable salt thereof for the manufacturing of a medicament for the treatment of a human subject suffering from cerebrovascular accident (CVA).

Without wishing to be bound by any theory, it is believed that afamelanotide or a pharmaceutically acceptable salt thereof benefits human subjects in terms of treatment symptoms and outcome for CVA, including higher survival rate and/or improved motor functioning and/or improved cognitive functioning and/or decreased ischemic, necrotic zones, in the short and/or longer term.

### Detailed description of the invention

According to the invention, we have surprisingly found that afamelanotide or a pharmaceutically acceptable salt thereof is effective in treatment of human subjects suffering from cerebrovascular accident (CVA).

For the purpose of this invention, the following abbreviations are used: Ala = alanine, Arg = arginine, Dab - 2,4-diaminobutyric acid, Dpr = 2,3- diaminopropionic acid, Glu = glutamic acid, Gly = glycine, His = histidine, HomoArg - homoarginine (one more -CH₂- unit in the alkyl chain than Arg), norArg - norarginine (one fewer -CH2 unit in the alkyl chain than Arg), Lys = lysine, Met = methionine, Nle = norleucine, Orn = ornithine, Phe = phenylalanine, (pNO2)Phe = paranitrophenylalanine, Plg = phenylglycine, Pro = proline, Ser = serine, Trp = tryptophan, TrpFor = N¹ formyl-tryptophan, Tyr = tyrosine, Val = valine.

For the purpose of this invention, all peptides are written with the N-terminus on the left and the C-terminus on the right; the prefix "D" before an amino acid designates the D-isomer configuration, and unless specifically designated otherwise, all amino acids are in the L-isomer configuration. All peptide and peptide derivatives are written with the acylated amino terminal end at the left, the N-terminus and the amidated carboxyl terminal at the right, the C-terminus. As will be understood, the acylated amino terminal end may be replaced by another group according to the invention but the orientation of the peptides and peptide derivatives remains the same. Following common convention, the first amino acid on the left is located at position 1, for instance, Nle (1) indicating that Nle is positioned at the N terminal end (on the left). The skilled person will understand that reference in this document to peptide molecules includes reference to derivatives thereof such as peptides that have been synthesized or comprise synthetic amino acids.

CVA (often called "stroke") is an acute and life-threatening medical condition. Causes for CVA can be ischemia (restricted blood supply) and hemorrhage (bleeding) in the brain. Sometimes TIA (Transient Ischemic Attack) may be included as separate cause of CVA. Ischemic CVA represents the majority of CVAs and is caused by obstruction of critical blood supply, for instance a blood clot occluding a cerebral artery. A hemorrhagic CVA occurs when a blood vessel in the brain ruptures, for instance due to high blood pressure, vascular malformations or tumor tissue. CVA may also be caused by trauma. Generally, CVA results in lack of oxygen and nutrients in the brain tissue and/or in damaged brain tissue in the vascularized area beyond the obstruction or hemorrhage.

The present invention is directed at treatment of CVA. The invention is further directed to treatment of CVA symptoms and/or causes; to reducing the complications of CVA; and/or to reducing the occurrence of complications of CVA.

Afamelanotide is a synthetic analogue of alpha-MSH and is also known by other names such as NDP-MSH, Melanotan I, CUV1647 and can be represented by the formula [Nle⁴, D-Phe⁷]-alpha-MSH. This means that the Nle replaces Met in the 4^{th} position and D-Phe replaces Phe in the 7^{th} position in natural alpha-MSH hormone.

For the purpose of this invention, afamelanotide may be used as such or in the form of a pharmaceutically acceptable salt thereof. Preferred examples of such salts are acetate, trifluoroacetate, sulfate, and chloride salts. The acetate salt is generally most preferred.

Afamelanotide or a pharmaceutically acceptable salt thereof is preferably administered to the human subject suffering from cerebrovascular accident (CVA) in a solution. Afamelanotide or a pharmaceutically acceptable salt thereof may for instance be administered as an infusion, an intramuscular injection, an intra-peritoneal injection or an subcutaneous injection or an injection in the cerebrum (intracerebral injection). Preferably, afamelanotide or a pharmaceutically acceptable salt thereof is administered as an injection, more preferably as an immediate release injection. Preferably, afamelanotide or a pharmaceutically acceptable salt thereof is injected subcutaneously.

Preferably, afamelanotide or a pharmaceutically acceptable salt thereof is administered with a dose of from 0.1 to 1mg/kg/day, wherein kg represents the weight of the human subject in kilograms. The preferred dose is preferably at least 0.01mg/kg/day, more preferably at least 0.25 mg/kg/day, most preferably at least 4 mg/kg/day and preferably up to 1 mg/kg/day, more preferably up to 0.85 mg/kg/day, most preferably up to 0.7, for instance 0.6 mg/kg/day.

Preferably, afamelanotide or a pharmaceutically acceptable salt thereof is administered at a dose of from 1 to 100mg. Preferably, the dose is at least 5mg, more preferably at least 10mg, most preferably at least 20mg, particularly preferred is at least 22mg and preferably up to 90mg, more preferably up to 70mg, most preferably up to 50mg, particularly preferred is up to 35mg. These doses also preferably represent the daily dose.

Afamelanotide or a pharmaceutically acceptable salt thereof is administered preferably at least once a day and preferably up to three times a day, more preferably once a day.

Preferably, afamelanotide or a pharmaceutically acceptable salt thereof is administered to the human subject for a period of at least 2 days, more preferably at least 5 days, most preferably at least 8 days. Preferably, afamelanotide or a pharmaceutically acceptable salt thereof is administered for a period of up to 50 days, more preferably up to 20 days and most preferably up to 12 days, for instance 10 days.

Preferably, afamelanotide or a pharmaceutically acceptable salt thereof is used to treat a human subject suffering from ischemic CVA or from CVA due to a hemorrhage.

Preferably, human subjects suffering from ischemic CVA are treated with a tissue plasminogen activator (tPA) and subsequently or prior with afamelanotide or a pharmaceutically acceptable salt thereof. Preferably, tPA is administered within 4.5 hours of the CVA event. Preferably, afamelanotide or a pharmaceutically acceptable salt thereof is administered thereafter. Preferably, afamelanotide or a pharmaceutically acceptable salt thereof is administered after 4.5 hours of the CVA event occurring.

Preferably, afamelanotide or a pharmaceutically acceptable salt thereof is administered as a composition. The composition is preferably liquid and preferably transparent. Afamelanotide is preferably included in the form of salt, preferably the acetate salt. Preferably, the composition comprises one or more pharmaceutically acceptable ingredients. Examples of pharmaceutically acceptable ingredients are water, salt, polymer, fatty acid, sugar, and surfactant. Preferably, the pharmaceutically acceptable ingredients include water and salt.

A further embodiment of the present invention relates to a method of treatment of human subjects suffering from cerebrovascular accident (CVA), wherein the human subject is treated with afamelanotide or a pharmaceutically acceptable salt thereof.

A further embodiment of the present invention relates to the use of afamelanotide or a pharmaceutically acceptable salt thereof for the manufacturing of a medicament for the treatment of a human suffering from cerebrovascular accident (CVA).

### Examples

Compositions comprising afamelanotide acetate were prepared and administered by injection on a daily basis to subjects diagnosed with cerebrovascular accident (CVA) at dosages indicated in the above description for periods of up to 10 or up to 20 days. Meanwhile neuroimaging such as CT-scanning, perfusion-CT, MRI and EEG techniques were used to monitor disease state, pathology, progression and recovery of the subjects, allowing for instance determination of ischemic, necrotic zones and cerebral functions. Motor functions are also subject of monitoring.

## Claims

1. Compound for use in the treatment of a human subject suffering from cerebrovascular accident (CVA) wherein the compound is afamelanotide or a pharmaceutically acceptable salt thereof.

2. Compound for use according to claim 1, wherein the compound is administered in the form of an injectable.

3. Compound for use according to any one of claims 1-2, wherein the compound is administered in an immediate or controlled release injection.

4. Compound for use according to any one of claims 1-3, wherein the compound is injected subcutaneously.

5. Compound for use according to any one of claims 1-4, wherein the compound is administered with a dose of from 0.1 to 1mg/kg/day for at least 1 day, wherein kg represents the weight of the human subject in kilograms.

6. Compound for use according to any one of claims 1-5, wherein the compound is administered at a dose of from 20 to 100mg.

7. Compound for use according to any one of claims 1-6, wherein the subject suffers from ischemic and/or hemorrhagic CVA.

8. Compound for use according to any one of claims 1-7, wherein the human subject is treated with a tissue plasminogen activator (tPA) and subsequently or prior treated with afamelanotide or a pharmaceutically acceptable salt thereof.

9. Composition comprising afamelanotide or a pharmaceutically acceptable salt thereof for use in treatment of a human subject suffering from a cerebrovascular accident (CVA).

10. Method of treatment of a human subject suffering from cerebrovascular accident (CVA), wherein the human subject is treated with afamelanotide or a pharmaceutically acceptable salt thereof.

11. Use of afamelanotide or a pharmaceutically acceptable salt thereof for the manufacturing of a medicament for the treatment of a human subject suffering from cerebrovascular accident (CVA).
